# EUROPEAN PATENT SPECIFICATION

(11) **EP 1 802 981 B1**
(45) Date of publication and mention of the grant of the patent: **11.04.2012**
(21) Application number: 05799285.1
(22) Date of filing: 21.10.2005
(51) Int. Cl.: G01N 33/68

(54) **EXPRESSION PROFILING PLATFORM TECHNOLOGY**
PLATTFORMTECHNOLOGIE ZUR EXPRESSIONSPROFILIERUNG
TECHNOLOGIE A PLATE-FORME DE PROFILAGE D'EXPRESSION

(30) Priority: 23.10.2004 US 621423 P
(43) Date of publication of application: 04.07.2007
(73) Proprietor: Biosystems Technology Corporation, F-91058 Evry Cédex (FR)
(72) Inventor: GUTTMAN, Andras, San Diego, CA 92130 (US); TAKACS, Laszlo, Newbury Park, CA 91320 (US)
(74) Representative: Becker, Philippe
(86) International application number: PCT/IB2005/003397
(87) International publication number: WO 2006/043179

(56) References cited:
- WO-A-2005/077106
- KOLLNER B ET AL: "Potential involvement of rainbow trout thrombocytes in immune functions: a study using a panel of monoclonal antibodies and RT-PCR" DEVELOPMENTAL & COMPARATIVE IMMUNOLOGY, vol. 28, no. 10, August 2004 (2004-08), pages 1049-1062, XP002367345 ISSN: 0145-305X
- SCRIVENER ELAINE ET AL: "Peptidomics: A new approach to affinity protein microarrays." PROTEOMICS, vol. 3, no. 2, February 2003 (2003-02), pages 122-128, XP002367346 ISSN: 1615-9853
- [Online] XP002367347 Retrieved from the Internet: URL:http://www.latince.com/op01-05.htm> [retrieved on 2006-02-12]

## Description

### Introduction

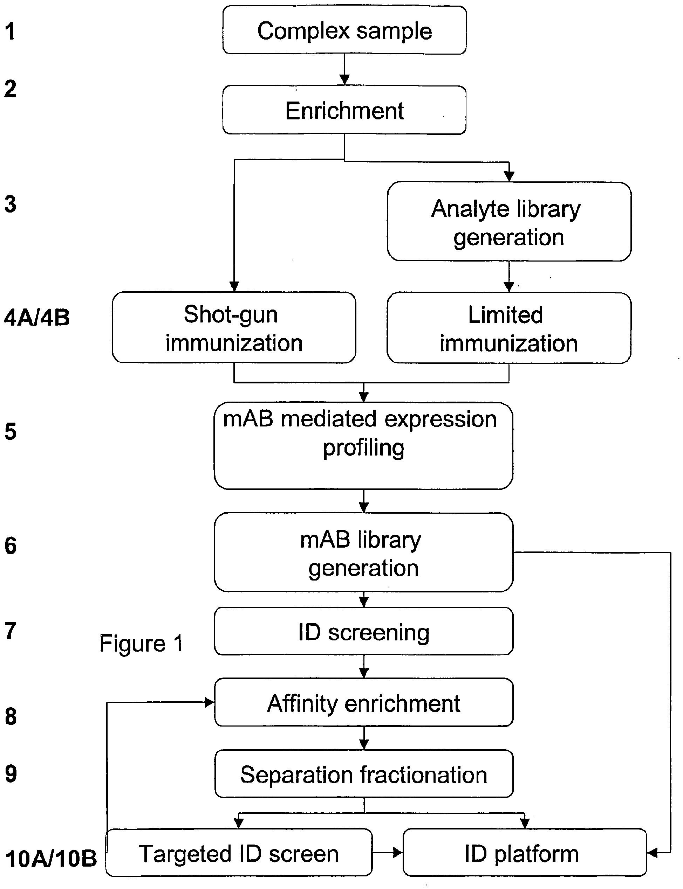

The present invention relates to an efficient mAb-based expression profiling technology platform (Fig.1) suitable for global and accurate measurement of proteins, peptides and metabolites in complex mixtures. The platform is comprised of new and well established technologies that are coupled in a unique fashion to provide a novel platform technology for (i) the discovery of differentially displayed elements or complex protein, peptide and metabolite mixtures and (ii) the development of robust mAb based assays that detect the differentially expressed elements.

Small molecule metabolite, peptide and protein expression analysis is a growing field in the medicinal, veterinarian, food and environmental monitoring and profiling areas. WO2005/077106 relates to a method of identifying biomarkers specific to a disease condition. Scrivener et al., 2003 concerns a peptidomic approach to affinity protein microarrays. Kollner et al., 2004, concerns a use of monoclonal antibodies for detecting cell-surface molecules on thrombocytes in order to investigate their fonctional properties. However, the prior art does not describe the process of specific immunization strategies, analyte library, antibody production, methods of producing monoclonal antibody panels suitable to monitor expression of metabolites and peptides, or the application of these to develop monoclonal antibody arrays. Furthermore, prior art does not disclose a global approach to the generation of antibody libraries.

Precise profiling with the method described herein provides an easy tool for the identification of relevant antigens as well as differences. Moreover, as parallel with the expression analysis and antigen ID, specific mAbs become available; simple or complex antibody based assays are designed for further monitoring and screening only the relevant and differentially expressed elements of the samples. The resulting mAb libraries with their antigen ID could serve as starting panel for the development of simplex or multiplex assays for sensitive, accurate and large scale measurements.

With specific immunization strategies, a large number of mono- or oligoclonal hybridoma supernatants are generated. The entire mAb panel covers the immunogen space of individual protein, peptide or metabolite epitope elements with at least one mAb. Thus, the advanced expression profiling technologies permit the construction of a platform that fulfills three yet unmet needs of protein and metabolite expression analysis : (i) quasi-global coverage, (ii) high level of reproducibility (iii) high sensitivity. Application of cutting edge protein, peptide and metabolite separation technologies for antigen ID completes the technology platform. The high throughput nature and global coverage enable the technology platform to feed into complex data analysis and integration processes.

### Summary of the Invention

The present invention relates to novel expression profiling methods suitable for global and accurate measurement of proteins, peptides and metabolites in complex mixtures. The invention can be used to generate libraries or panels of monoclonal antibodies specific for blood antigens. It may be used to determine the expression of any analyte from a large variety of complex samples, including samples of human, animal, vegetal or environmental origin. The invention is useful to provide markers, targets, diagnostics and tools useful e.g., in medicinal, veterinarian, food and environmental industries.

The object of this invention relates to a method as defined in the claims.

This invention also discloses a method of monoclonal antibody mediated expression profiling by the generation of many mono or oligoclonal hybridomas, mono or oligoclonal hybridoma supernatants, monoclonal antibodies, a monoclonal antibody panel from nom human vertebrates immunized as discussed above and screening said monoclonal antibody panel for differential reactivity for at least two different samples of complex analytes in a process that involves the following steps. In a particular embodiment, the method further comprises a step of identifying antigens recognized by antibodies or derivatives thereof within said complex sample and/or analyte library, said identification typically comprising the steps below:
- Reacting selected monoclonal antibody with complex analyte or element of analyte library, or with a biological sample
- Eluting cognate antigen
- Identification of cognate antigen via hyphenated chromatography and/or electric field mediated separation process - mass spectrometry or direct peptide sequencing methods.

The complex analyte sample may be any complex sample of biological, environmental, industrial, etc. origin, such as a mixture of proteins and small molecules, e.g.: biological samples like: plasma, serum, urine, body fluids, cell lysates, tissue extracts of human and animal origin. Environmental samples; such as soil, water, cloud condensate, food processing intermediates and food products. Cosmetics and other healthcare products. Any complex mixture that contains immunogen metabolites and/or immunogen proteins, peptides. The complex sample could be a mix of individual complex analyte samples.

In a particular embodiment of the invention, the sample is conjugated to a carrier prior to immunization. In this respect, the invention also discloses a method for producing a panel of monoclonal antibodies, comprising the steps of:
a) providing at least one complex analyte sample comprising antigens;
b) partitioning the whole or any part of the analyte library, where parts share physicochemical or biological properties,
c) binding, preferably covalently, library members to a carrier (e.g, a protein) such as, without limitation, albumin, polylysin, keyhole limplet haemocyanin, etc.,
d) using conjugated analyte library member as an immunogen to immunize a non-human vertebrate, referred to as limited immunization; and
e) preparing monoclonal antibodies, or derivatives thereof, specific for complex analyte sample antigens, from said immunized non-human mammalian, thereby obtaining said monoclonal antibody panel.

In a preferred embodiment, the method further comprises the following steps:
f) profile gene expression at the peptidome or metabolome level by the set of monoclonal antibodies, or derivatives thereof;
g) antigen ID screening of analyte library elements
h) affinity enrichment of antigens of interest by means of using monoclonal antibodies or derivatives thereof for highly specific recognition;
i) treating affinity enriched sample to fractionate and identify elements of interest; and identify elements of interest

The invention also discloses a method for producing a library or panel of monoclonal antibodies, or derivatives thereof, specific for human blood antigens, the method comprising the steps of:
a) providing at least one biological sample comprising human blood antigens;
b) treating said sample under conditions allowing depletion of abundant proteins while retaining low abundant proteins present in normal human blood;
c) using said treated sample or a portion thereof as an immunogen to immunize a non-human mammalian; and
d) preparing monoclonal antibodies, or derivatives thereof, specific for human blood antigens, from said immunized non-human mammalian, thereby obtaining said mAb panel.

The invention also provides a mAb panel obtainable by such a method, as well as to any uses thereof.

### Legend to the Figures

Figure 1: Shotgun immunization based protein, peptide and metabolite expression profiling platform
Figure 2: SDS PAGE electrophoresis of treated human plasma analyte, 5 ug of complex protein mix was loaded to each well, a 4-20% acrylamide gel was run and stained with silver nitrate.
Figure 3: Monoclonal antibodies are captured in the ELISA assay by a goat anti Ig-Fc antibody. Captured antibodies are incubated with biotinylated plasma samples. Reaction is developed by peroxidase coupled avidin complexes. Peroxidase reaction is visualized via OPD and the reaction is measured at 492 nm in an ELISA reader. To detect individual proteins a direct ELISA reaction was applied on pure target proteins and on the eluate of the Aglient column.
Figure 4: Comassie blue stained SDS PAGE gel. Numbers in parenthesis show the sequence coverage obtained by MALSI-TOF MS/MS.

### Detailed description of the invention

### The platform:

The invention discloses a method of global protein peptide and/or metabolite expression profiling from a complex analyte sample, comprising of the following steps:
a) Enrichment of the complex analyte sample ;
b) Immunization of a non-human mammalian subject with either (i) the enriched complex analyte sample or an aliquot or dilution thereof (shotgun immunization) or (ii) or with a limited analyte library prepared from the enriched complex analyte sample (Limited Immunization) ; or (iii) with the analyte conjugated to a carrier prior to immunization (Conjugated immunization) and
c) Generation of a panel of monoclonal antibodies, derivatives thereof, corresponding hybridomas and/or producing cells, and optionally, analysing the expression profile of said antibodies and/or identifying any antigen bound by antibodies from the panel.

The non human vertebrate may be any non human mammal, such as but not limited to a rodent, a rabbit, or a chicken.

The invention also discloses a method of monoclonal antibody mediated expression profiling by the generation of many mono or oligoclonal hybridomas, mono or oligoclonal hybridoma supernatants, monoclonal antibodies, a monoclonal antibody panel from nom human vertebrates immunized as disclosed above and screening said monoclonal antibody panel for differential reactivity for at least two different samples of complex analytes. The process preferably involves the following steps:
- Generation of a panel of monoclonal antibodies or derivatives thereof, wherein said panel comprises a plurality of containers comprising annotated monoclonal antibodies, corresponding to monoclonal or oligoclonal antibody producing hybridomas, specific for distinct analyte elements, e.g. human blood antigens. Moreover, wherein said panel comprises antibodies, or derivatives thereof, those e.g., which bind to low abundant antigens from diseased and from healthy human subjects.

The complex analyte preferably comprises of at least two clinical samples. The clinical samples may represent at least two disease conditions or at least one drug-responding group and one non-responding group or disease susceptible and non-susceptible individuals or diseased and apparently healthy subjects such as but not limited to cancer patients before and after tumor resection or cancer patients with and without recurrence of primary cancer, or cancer patients with and without metastasis.

According to specific embodiments, the clinical sample is selected from human serum or plasma, human urine, human sputum, human brochoalveolar fluid, human biopsy material, human tissue section, human faeces and human exudates.

In a specific embodiment, the monoclonal antibody panel is screened via ELISA assay.

In a further specific embodiment, the monoclonal antibody panel is immobilized to a surface, such as a solid surface, in particular but not limited to glass, silicon, plastic, membrane (and is screened e.g., as microarray). Alternatively, the monoclonal antibody panel may be immobilized to any gel for screening. The monoclonal antibody panel may be screened in multiplex antibody arrays comprising fluorescent antibody conjugated beads. In a particular embodiment, the monoclonal antibody panel is screened via chemiluminescent assay.

The density of the array may be variable, and adjusted by the skilled artisan. Typical densities include a density ranging from 10-1,000/ cm² to about 1,000-1,000,000/ cm².

### 1. Complex samples;

Mixture of proteins and small molecules, e.g., but not limited to: biological samples like: plasma, serum, urine, body fluids, cell lysates, tissue extracts of human and animal origin. Environmental samples; such as soil, water, cloud condensate, food processing intermediates and food products. Cosmetics and other healthcare products. Any complex mixture that contains immunogen metabolites and/or immunogen proteins, peptides. The complex sample could be a mix of individual complex analyte samples.

In order to enrich for differentially expressed elements of two or multiple complex samples to be compared; the samples will be enriched for the those elements that are of special interest (see below)

### 2. Enrichment by partitioning

Some elements of complex samples are irrelevant for subsequent analysis, either because these do not carry information relative to the question that drives the analysis, and/or their abundance is so high that it interferes and jeopardizes the analysis process (e.g. albumin in human plasma samples). Affinity chromatography will be used to eliminate these elements. The partitioning process may be mediated by individual monoclonal antibodies, mixtures of monoclonal antibodies, polyclonal antiserum or ligands to which undesired elements will bind. Either the depleted fraction or the eluate of affinity chromatography process might be used for further steps.

A specific example: Immunosorbent chromatography, e.g.: columns from GenWay, Inc or Agilent Inc. are used to remove the most abundant serum and plasma proteins. Alternatively, anti-human serum could be used to remove elements of the complex human mixture that are both relatively highly abundant, and present or enriched only in healthy individuals. Moreover, polyclonal antisera prepared to specific mixtures or proteins or metabolites, metabolite classes could be applied either to enrich or to deplete these from the complex analyte mixture.

In a specific embodiment, the enrichment is carried out by treating the sample to partition. Preferably, the treating comprises separation technology ; affinity enrichment e.g., using antibodies ; organic ligand affinity chromatography ; ion exchange chromatography ; hydrophobic interaction chromatography ; hydrophilic interaction chromatography ; electrophoresis ; size exclusion chromatography; chromatofocusing ; isoelectrofocusing or a combination thereof.

### 3. Analyte library generation

### (Multidimensional separations based on physical, chemical and biochemical characteristics, differential display)

In order to support downstream analyte identification (ID) processes, complex mixtures are separated into specific classes via a suitable multidimensional and hierarchic separation process that is based on physical, chemical and biochemical characteristics of the complex mixture. The result of this step is a hierarchic set of pools; within the pool of complex mixtures the individual elements share at least one common characteristics. Process proximal pools differ in complexity from process distal pools and share fewer characteristics. Process ultimate pools might contain only a single type/class of element that is apparently homogeneous and contains no or only trace amount of less related contaminating elements. These ultimate pools if loaded to identification process (e.g. mass spectrometry based protein ID) will provide a single ID or a very likely one.

Screening of pools with mAb-s (ID screen) provides information on the shared physical, chemical and biochemical characteristics.

Analyte library is not necessarily the same as the one that is being profiled.

Differential display analysis of labeled samples from the separated fractions:
Comparison of analyte libraries allows the identification of apparent differences at the level of pools in complexity levels and relative representation of individual elements. These pools being differentially displayed, could be applied preferentially for the immunization process (Limited immunization).

For limited immunization, an analyte library is generated, typically by separating analytes having common physicochemical or biological properties. Typically, all elements recovered and placed to individual containers.

### 4. 4.A. Shot-gun immunization

Immunization with complex protein sample: Either the enriched fraction or the complex protein mixture is used to immunize mice. Immunization is done by the use of well established technologies.

### 4.B. Limited immunization

Immunization with analyte pools. To increase the chances of obtaining monoclonal hybridomas reactive with all immunogen elements, lower complexity analyte pools are used for immunization. These pools could contain proteins, peptides or metabolites that share at least one important physical chemical or biochemical characteristics. As described in 3A, proteins are used for immunization directly, while peptides, and metabolites are used after derivatization to adjuvant immunogens.

In a specific embodiment of limited immunization, non human mammals are immunized with a one or more elements of the analyte library.
In particular embodiment, more than two elements of the analyte library share at least one physicochemical characteristic and/or at least one biochemical characteristic and/or at least one immunochemical property and/or at least one affinity binding capacity and/or are homologous in their peptide sequence.

### 4.C. Conjugated immunization

Immunization with complex peptide mixtures or complex metabolites, or individual peptide or metabolites: This step involves derivatization of an adjuvant immunogen carrier protein or artificial adjuvant immunogenic polymer in fashion that permits conjugation of peptides or metabolites. Metabolites and peptides are coupled to the adjuvant immunogen via their reactive groups in separate conjugation processes, e.g.: one process for OH esters, another for NH2 groups etc.. Finally derivatized adjuvant immunogens are mixed and the mixture is used to immunize mice

### 5. Monoclonal antibody (mAb) mediated expression profiling of individual samples

High sensitivity micro-ELISA assays are designed that use the monoclonal hybridoma supernatant and labeled complex tracers derived from the complex sample or its pools. Thousands of mAb containing hybridoma supernatants are tested in a screen to identify those that discriminate individual classes of analyte samples (e.g. derived from disease vs. healthy individuals, or treated vs. non treated groups, etc.).

### 6. Monoclonal antibody panel generation

After rigorous statistical calculations a panel of mAb containing hybrodomas are selected. Large scale mAb generation could be initiated for each selected hybridoma at this step. The panel is subjected to downstream steps in order to identify "ID" each immunogen antigen that reacts with an individual mAb in panel.

The antibody derivatives may be an antibody fragment, preferably selected from Fab, Fab', CDR, and single chain antibodies (ScFv). The antibody derivative is preferably a human or humanized antibody or fragment thereof. Such derivatives may be produced by any method known per se in the art.
One way to produce humanized antibodies is to isolate the cDNA of a particular mouse monoclonal antibody, sequence the cDNA region coding for the peptide region that binds to the antigen. One can directly sequence this region via peptides sequencing technologies. In the next step the region is cloned into the similar region of a human antibody cDNA and expressed. Particular care should be paid to engineer the regions of glycosilation to ensure that these are human like. The step above can be applied to the heavy chain or to the light chain or to both. An alternative way is to produce monoclonal antibodies from transgenic mice that cany a part of or the entire human antibody gene sets, but may not have mouse antibody genes. These mice produce human antibodies and may not produce mouse antibodies

### 7. ID screen

This step (optional) involves the screening of analyte library pools in a hierarchic and economic manner to identify the pool that contains the antigen recognized by the monoclonal antibody, or the monoclonal antibody containing hybridoma supernatant. If necessary, affinity enrichment and targeted screening steps are deployed to identify the antigen.

### 8. Affinity enrichment

Affinity enrichment that can be but not limited to column or microbead based processes. The relevant mAb's generated during steps 1-5 and screened positive in Step 6 are immobilized to appropriate stationary phase material or micro-bead substrate and used as bait for antigen purification. This process can be repeated as many times as necessary to collect the required amount of material for downstream processing.

### 9. Separation and fractionation

The separation and fractionation of the enriched eluent from Step 8 can be accomplished but not limited to liquid chromatography (LC), capillary electrophoresis (CE), capillary electrochromatography (CEC), microchip based analytical methods or other separation technologies. The collected fractions or split eluent stream is being interrogated in Step 10 for activity in a mAb mediated screen (targeted screening)

### 10. Targeted screening

(i) (10A) One part of the split flow is used for screening the fractions of Step 8 for antibody reactivity with the appropriate mAb, and collected for ID analysis in step 10B.
(ii) (Loop to Step 8) Break the mAb/AG complex and reprocess the mAb in Step 8.
(iii)(10B) Mass spectrometry (MS and MS") based identification using but not limited to ESI or MALDI ionization methods. In case of proteins, digestion, separation and MS". In case of metabolites, separation and MS".

In a particular embodiment, the method of ID screening comprises a step of identifying antigens recognized by antibodies or derivatives thereof within said complex sample and/or analyte library where identification comprises the steps below:
- Reacting selected monoclonal antibody with complex analyte or element of analyte library, or with a biological sample
- Eluting cognate antigen
- Identification of cognate antigen via hyphenated chromatography and/or electric field mediated separation system - mass spectrometry or direct peptide sequencing methods.

The identification of said antigens typically comprise contacting an antibody or derivative thereof with a biological sample and determining the identity of an antigen specifically bound to said antibody or derivative thereof. Identification of the cognate antigen may be preceded by screening the entire or part of the analyte library in order to identify the source of material for the cognate antigen ID. Alternatively, or in addition, identification of the cognate antigen may be preceded by affinity enrichment of the antigen and/or by partitioning, including but not limited to separation and fractionation.

In a particular embodiment, the process is an automated platform, and/or involves one or more microfluidic or micro total analysis system (µTAS) chip

### Production of a panel (or library) of antibodies specific for human blood antigens

The invention also discloses a method for producing a library or panel of monoclonal antibodies, or derivatives thereof, specific for human blood antigens, the method comprising the steps of:
a) providing at least one biological sample comprising human blood antigens;
b) treating said sample under conditions allowing depletion of abundant proteins while retaining low abundant proteins present in normal human blood;
c) using said treated sample or a portion thereof as an immunogen to immunize a non-human mammalian; and
d) preparing monoclonal antibodies, or derivatives thereof, specific for human blood antigens, from said immunized non-human mammalian, thereby obtaining said panel (or library).

According to a preferred embodiment, the method further comprises a step of profiling antibodies, or derivatives thereof, within the panel against one or several control or diseased samples, to obtain an annotated panel of monoclonal antibodies, or derivatives thereof. The profiling step typically comprises determining whether the antibody or derivative thereof specifically binds an antigen contained in a blood sample from a control or diseased human subject.

In a particular embodiment, the profiling step comprises determining whether the antibody or derivative thereof binds an antigen present in at least one control sample and two diseased samples.

The method of this invention preferably further comprises a step of identifying antigens recognized by antibodies or derivatives thereof within said panel. The identification of said antigens typically comprises contacting an antibody or derivative thereof from the panel with a biological sample and determining the identity of an antigen specifically bound to said antibody or derivative thereof.

As discussed above, the antibody derivative is e.g., an antibody fragment, preferably selected from Fab, Fab', CDR and single chain antibodies (ScFv). The antibody derivative is preferably a human or humanized antibody or a fragment thereof.

The biological sample comprising human blood antigens typically is or derives from a human plasma sample, a human serum sample or a human blood sample. The biological sample is preferably derived from a healthy human subject.

In a particular embodiment of the method, the panel comprises monoclonal antibodies or derivatives thereof produced from biological samples from different human subjects. The biological samples may all derive from several healthy subjects, or from several healthy and diseased subjects.

In step b) above, the sample is typically contacted with an affinity column that removes from 2 to 22 most abundant human proteins. The depleted sample preferably comprises between about 5 to 10% of total human serum proteins.

In step c) above, the whole treated sample, in aliquots, may be used as an immunogen, or different classes of antigens present in the sample are separated, and separate immunizations are performed with each of said classes.

The panel can comprise a plurality of monoclonal antibodies, producing hybridomas and/or derivatives thereof, which are arranged in separate containers. In this respect, a particular object of this invention also resides in a panel (or library) of monoclonal antibodies, or derivatives thereof, wherein said panel comprises a plurality of containers comprising annotated monoclonal antibodies, or derivatives thereof, or corresponding producing hybridomas, specific for distinct human blood antigens, wherein said panel comprises antibodies, or derivatives thereof, that bind low abundant antigens from diseased and from healthy human subjects.

The invention also discloses a product comprising, immobilized on a support, preferably in an ordered manner, a plurality of monoclonal antibodies, or derivatives thereof, specific for distinct human blood antigens, wherein said product comprises antibodies, or derivatives thereof, that bind low abundant antigens from diseased and from healthy human subjects. As discussed above, the support may be a solid or semi-solid material, such as a membrane, glass, plastic, ceramic or metal support having a surface, or a gel.

Such a panel of mAbsor product may be used to identify markers, therapeutic antibodies, to design diagnostic kits, etc. In this respect, a particular object of this invention also concerns a method for identifying antibodies that bind a selected target, the method comprising contacting said target with all or a portion of a panel of monoclonal antibodies, or derivatives thereof, as defined above or obtainable by a method as defined above, or with a product as defined above, under conditions allowing an antigen-antibody reaction to occur, and identifying one or several monoclonal antibodies, or derivatives thereof, from said mAb panel, that bind said target.

The invention also discloses a method for identifying antibodies that are specific for a particular condition or disease, the method comprising contacting a biological sample from a mammalian having said condition or disease with all or a portion of a panel of monoclonal antibodies, or derivatives thereof, as defined above or obtainable by a method as defined above, or with a product as defined above, under conditions allowing an antigen-antibody reaction to occur, and identifying one or several monoclonal antibodies, or derivatives thereof, from said library, that form antiboby-antigen complexes with said sample.

The invention further relates to a method for identifying one or several mammalian antigens specific to a condition or disease, the method comprising contacting a biological sample from a mammalian having said condition or disease with all or a portion of a panel of monoclonal antibodies, or derivatives thereof, as defined above or obtainable by a method as defined above, or with a product as defined above, under conditions allowing an antigen-antibody reaction to occur, identifying one or several monoclonal antibodies, or derivatives thereof, from said panel, that form antiboby-antigen complexes with said sample and, identifying antigens engaged into said complexes.

Further aspects and advantages of the present invention will be disclosed in the following examples, which should be considered as illustrative and not limiting the scope of this application.

### Examples

### I-Enrichment by partitioning

A complex analyte sample (human plasma) is treated to partition. Highly abundant proteins are separated here from medium and low abundant ones via affinity chromatography using a commercial chromatography system with a multiaffinity removal column (Agilent). Highly abundant proteins are those that are represent in the plasma at a concentration level that is higher than I mg/ml. The experiment is performed as described in the Agilent Technologies manual. In the example, the enriched sample contains minimal, trace, or not detectable concentrations of the following proteins: human serum albumin, IgA, haptoglobin, anti-trypsin, IgG, transferrin

To test the efficiency of enrichment, specific protein analytes were coated onto plastic plates and specific mAbs were used to detect the bound analyte species (e.g. Apolipoprotien Al, complement factor C3, IgA). The amount of specific mAb bound is in direct correlation with the analytes species quantity bound to the plastic surface, mAb binding was visualized by horse radish peroxidase coupled rabbit anti-mouse Ig. Standard curves were used to calculate relative abundance level, which is expressed in fractions (%) of total protein content of the analyte. Results show that only traces of IgA is detectable, while other analytes species are enriched.

**Table 1. Relative abundance level of various protein analyte species before and after the treatment.**

| | **Before Depletion (%)** | **After depletion (%)** |
|---|---|---|
| **ApoA1** | 1.1 | 16.5 |
| **C3** | 0.5 | 20 |
| **IgA** | 1.25 | 0.32 |

### II-Analyte library generation

Analyte libraries are generated from complex analyte mixture that may contain proteins, pcptidcs and/or metabolites at the same time. Multidimensional separation technology is applied to partition all elements or specific classes of elements, e.g. proteins from all metabolites. Moreover, complex separation processes can also be applied e.g. those that retain the proteins in their antigenic conformations, yet allow separation of classes or individual types of proteins. Separation technologies that use caotripic agents e.g, detergents, e.g. SDS are usually irreversibly denaturing, prevent the analyte to react with antibodies that exclusively recognize the natural conformation. High concentration of organic solvents and the process of binding and elution from separation surfaces are denaturing as well. Thus a carefully selected process is applied that conserves antigenic determinants. An initial step could be affinity chromatography with polyclonal antibody directed against components of the complex analyte.

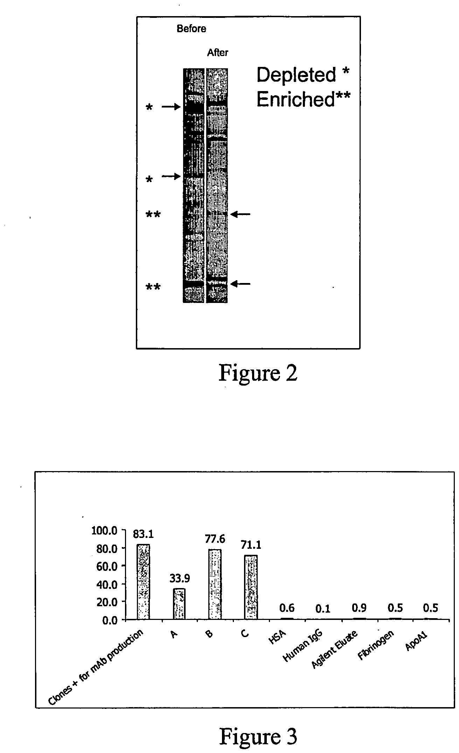

To determine the efficiency and progress of analyte library preparation, SDS PAGE electrophoresis separation was done on samples that have undergone affinity chromatography separation mediated by a polyclonal antibody directed to the human serum. The separation step clearly enriches some elements while some others are virtually eliminated. (Fig. 2).

### III-Shotgun immunization, mAb mediated expression profiling

Complex enriched or enriched and treated analyte mix diretly (protein samples) or after conjugation en-mass to immunoadjuvant carriers (peptide and metabolite samples) are injected into mice or other species that develops a clonable antibody repertoire. In the example, treated complex analyte mix, human plasma, was injected into Balbc female mice in the presence of complete Freund adjuvant first, then bi-weekly in the presence of incomplete adjuvant. Injections were done into the footpad and s.c. at multiple places. The last injection was done i.v. without adjuvant. Three days after the last injection spleen cells were fused to Sp2Ag0 mouse hybridoma partner cells in the presence of 50% Polyethylene glycol. Fused cells were cultured in 96 well plates in the presence of selection medium. One thousand one hundred eighty five hybridoma supernatants from this antibody panel were screened in ELISA assays, As shown on Figure 3. 83.1% of the supernatants produced antibodies. Three different plasma preparations have been tested. It is evident, that 77.6 percent of the clones react with plasma proteins in sample A, only 33.9 in sample B, while 71.1 % react with sample C, 14.5% of clones positive with B, give higher signal with C than B while 75.1% of clones positive with sample A give higher signal with B than A. The majority of the mAB panel elements thus detect representational differences in protein level and together this is suitable for protein expression profiling. Utility of a plasma proteome specific mAb panel or library is dependent on the level of redundancy, e.g. the number of antibody clones directed against the most abundant proteins in the plasma. We have tested some of those that are removed from the complex analyte by the analyte treatment method described in example-I. Results are shown for serum albumin, IgG, fibrinogen and apolipoprotein A1 and the eluate of the Agilent depletion column (Fig. 3). Clones specific for these abundant proteins are represented at <1 % frequency.

### IV- Affinity enrichment, separation fractionation, ID screening.

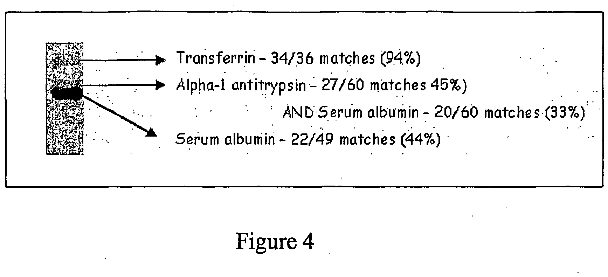

One possible regimen for protein ID is affinity purification followed by SDS gel electrophoresis and mass spectrometry (MS) on material derived from gel slices containing a single stainable band. In Figure 4 we show that apparent purity (single band one gel slice) is sufficient criteria for good quality protein ID. In the complex analyte sample we applied onto SDS PAGE electrophoresis multiple proteins were present, still bands cut out from the gel provided single proteins and high quality ID, as analyzed by MALDI-TOF MS technology. As described below: Gel bands were rinsed with an ammonium bicarbonate buffer/acetonitrile 1:1 mixture in order to eliminate the gel stain (CBB) and SDS. The proteins were reduced by DTT, the free sulfhydrils were derivatized with iodoacetamide. Then the proteins were incubated with side chain protected porcine trypsin (Promega) for 4 hrs at 37°C. The resulting peptides were extracted and zip-tip purified. The unfractionated digests were subjected to MALDI-TOF MS analysis using 2,5-dihydroxy-benzoic acid (DHB) as matrix. An MS-Fit (http://prospector.ucsf.edu ) database search was performed with the masses detected against the NCBI nonredundant protein database NCBInr.2005.01.06. During the search no species restriction was applied. The identity of the proteins was verified by post source decay (PSD) analysis of a selected peptide. The protein MWs and pIs obtained represented the full length sequences as listed in the database-that do not necessarily reflect the size of the expressed, especially the processed, mature, and active proteins.

## Claims

1. A method of identifying human blood antigens in an analyte biological sample, comprising the following steps:
a) enrichment of the analyte sample, wherein the sample is treated to partition using a separation technology under conditions allowing depletion of at least 2 to 22 most abundant human proteins represented in the plasma at a concentration level higher than 1 mg/ml, while retaining low abundant proteins present in normal human blood, and wherein the depleted sample comprises less than 5 % of total human serum proteins;
b) optionally, separation of the enriched analyte sample into specific classes (pools) of analytes each class (pool) of analytes containing elements sharing at least one common physical, chemical, immunochemical or biochemical characteristic;
c) immunization of a non-human mammalian subject with either (i) the enriched analyte sample of step a) or an aliquot or dilution thereof or (ii) with a pool of analytes of step b) ;
d) generation of a panel of monoclonal antibodies, derivatives thereof or corresponding hybridoma cells from said immunized non-human mammalian subject;
e) selecting antibodies of d) which bind antigens of said analyte sample;
f) purifying antigens from the analyte sample by affinity enrichment using monoclonal antibodies or derivatives thereof, selected in e); and
g) determining the identity of said human antigens.

2. A method of claim 1, wherein said treatment of step a) comprises organic ligand affinity chromatography, ion exchange chromatography, hydrophobic interaction chromatography, hydrophilic interaction chromatography, electrophoresis, size exclusion chromatography, Chromatofocusing, or isoelectrofocusing, or a combination thereof.

3. A method of any one of the preceding claims, wherein the non human mammal is a rodent or a rabbit.

4. A method of any one of the preceding claims, wherein the elements of the pool of analytes of step b) share affinity binding capacity, and/or are homologous in their peptide sequence.

5. A method of any one of the preceding claims, wherein step e) comprises contacting said panel of monoclonal antibodies, or derivatives thereof, as defined or obtainable by a method of any one of claims 1-4, with said analyte sample, under conditions allowing an antigen-antibody reaction to occur, and identifying one or several monoclonal antibodies, or derivatives thereof, from said panel, that bind said analyte sample.

6. A method of any one of the preceding claims, wherein step e) comprises identifying antibodies that are specific for a particular condition or disease, the method comprising contacting an analyte biological sample from a mammalian having said condition or disease with all or a portion of a panel of monoclonal antibodies, or derivatives thereof, as defined or obtainable by a method of any one of claims 1-4, under conditions allowing an antigen-antibody reaction to occur, and identifying one or several monoclonal antibodies, or derivatives thereof, from said panel, that form antibody-antigen complexes with said sample.

7. A method of any one of claims 1 to 6, wherein said monoclonal antibody panel is screened via ELISA assay.

8. A method of any one of claims 1 to 7, wherein said monoclonal antibody panel is immobilized to a solid surface, preferably a glass surface, silicon surface, plastic surface or metal surface, and screened as microarray, or wherein said monoclonal antibody panel is immobilized to any gel, or membrane, preferably lipid membranes, for screening.

9. A method of one of claims 1 to 8, wherein said monoclonal antibody panel is screened in multiplex antibody arrays comprising fluorescent antibody conjugated beads, or via chemiluminescent assay, or via assays that do not use label, preferably mass spectrometry.

10. A method of any one of claims 8 to 9, wherein the density of the array is between 10-1,000/cm², or between 1,000-1,000,000/cm².

11. The method of any one of the preceding claims, wherein the identity of cognate antigen specifically bound to said antibody, or derivative thereof, is determined via hyphenated chromatography and/or electric field mediated separation methods - mass spectrometry or direct peptide sequencing methods.

12. The method of any one of the preceding claims, wherein the analyte biological sample is selected from the group consisting of plasma, serum, urine, body fluids, cell lysates, tissue extracts of human origin ; any mixture that contains immunogen metabolites and/or immunogen proteins or peptides ; or a combination thereof.

13. A method of any one of the preceding claims, wherein the process involves one or more microfluidic chip(s) or micro total analysis system (µTAS).

14. A method of any one of the preceding claims, wherein, prior to immunization, the analyte is conjugated to a carrier, preferably albumin, polylysin or keyhole limplet haemocyanin.

15. The method of any one of the preceding claims, wherein the antibody derivative is an antibody fragment, preferably selected from Fab, Fab', CDR and single chain antibodies (ScFv), further preferably is a human or humanized antibody or fragment thereof.

16. The method of any one of the preceding claims, wherein the analyte biological sample is derived from a healthy human subject.

17. The method of any one of the preceding claims, wherein the panel comprises monoclonal antibodies or derivatives thereof produced from analyte biological samples from different human subjects, preferably from several healthy subjects or from several healthy and diseased subjects.

18. The method of any one of the preceding claims, wherein different classes of antigens present in the sample are separated, and separate immunizations are performed with each of said classes.

19. The method of any one of the preceding claims, wherein the panel comprises a plurality of monoclonal antibodies, producing hybridomas and/or derivatives thereof, which are arranged in separate containers.

## Patentansprüche

1. Ein Verfahren zur Identifizierung von humanen Blutantigenen in einer biologischen Analyt-Probe, umfassend die folgenden Schritte:
a) Anreicherung der Analyt-Probe, wobei die Probe zur Trennung unter Verwendung einer Trennungstechnologie unter Bedingungen, die einen Abbau von mindestens 2 bis 22 der häufigsten humanen Proteine, die im Plasma in einem Konzentrationsgrad höher als 1 mg/ml auftreten, behandelt wurde, während weniger häufige Proteine die in normalem humanen Blut vorkommen, beibehalten werden und wobei die abgebaute Probe weniger als 5 % der gesamten humanen Serumproteine umfasst,
b) optional, Abtrennung der angereicherten Analyt-Probe in spezifische Klassen (Pools) von Analyten, wobei jede Klasse (Pool) von Analyten Elemente enthält, die mindestens eine gemeinsame physikalische, chemische, immunochemische oder biochemische Eigenschaft teilen,
c) Immunisierung eines nicht-humanen Säugersubjekts mit entweder (i) der angereicherten Analyt-Probe aus Schritt a) oder einem Aliquot oder Verdünnung davon oder (ii) mit einem Pool von Analyten aus Schritt b),
d) Generieren eines Panels monoklonaler Antikörper, Derivate davon oder entsprechender Hybidoma Zellen aus dem immunisierten nicht-humanen Säugersubj ekt,
e) Auswahl von Antikörpern aus d), die Antigene der Analyt-Probe binden,
f) Aufreinigung von Antigenen aus der Analyt-Probe durch Affinitätsanreicherung unter Verwendung monoklonaler Antikörper oder Derivaten davon, ausgewählt in e) und
g) Bestimmung der Identität der humanen Antigene.

2. Ein Verfahren nach Anspruch 1, wobei die Behandlung von Schritt a) organische Liganden Affinitätschromatographie, Ionen-Austausch-Chromatographie, hydrophobe Interaktionschromatographie, hydrophile Interaktionschromatographie, Elektrophorese, Größenausschlusschromatographie, Chromatofokussierung oder Isoelektrofokussierung oder eine Kombination davon umfasst.

3. Ein Verfahren nach einem der vorhergehenden Ansprüche, wobei der nicht-humane Säuger ein Nager oder ein Hase ist.

4. Ein Verfahren nach einem der vorhergehenden Ansprüche, wobei die Elemente des Pools der Analyten von Schritt b) Affinitätbindungskapazität teilen und/oder homolog in ihrer Peptidsequenz sind.

5. Ein Verfahren nach einem der vorhergehenden Ansprüche, wobei Schritt e) das Kontaktieren des Panels monoklonaler Antikörper oder Derivaten davon, wie durch ein Verfahren nach einem der Ansprüche 1-4 definiert oder erhältlich, mit der Analyt-Probe, unter Bedingungen, die eine Antigen-Antikörper Reaktion ermöglichen, und die Identifizierung einer oder mehrerer monoklonaler Antikörper oder Derivaten davon von dem Panel, das die Analyt-Probe bindet, umfasst.

6. Ein Verfahren nach einem der vorhergehenden Ansprüche, wobei Schritt e) die Identifizierung von Antikörpern, die spezifisch für einen bestimmten Zustand oder eine Erkrankung sind, umfasst, wobei das Verfahren das Kontaktieren einer biologischen Analyt-Probe von einem Säuger, der den Zustand oder die Erkrankung hat, mit allen oder einem Teil eines Panels monoklonaler Antikörper oder Derivaten davon, wie durch ein Verfahren nach einem der Ansprüche 1-4 definiert oder erhältlich, unter Bedingungen, die eine Antigen-Antikörper Reaktion ermöglichen, und die Identifizierung einer oder mehrerer monoklonaler Antikörper oder Derivaten davon von dem Panel, die Antikörper-Antigen Komplexe mit der Probe bilden, umfasst.

7. Ein Verfahren nach einem der Ansprüche 1 bis 6, wobei das Panel monoklonaler Antikörper per ELISA Nachweis durchmustert wird.

8. Ein Verfahren nach einem der Ansprüche 1 bis 7, wobei das Panel monoklonaler Antiköper auf einer festen Oberfläche, bevorzugt einer Glasoberfläche, Silikonoberfläche, Plastikoberfläche oder Metalloberfläche immobilisiert wird und als Mikroarray gerastert wird oder wobei das monoklonale Antikörper Panel auf irgendeinem Gel oder Membran, bevorzugt Lipidmembranen zur Durchmusterung immobilisiert wird.

9. Ein Verfahren nach einem der Ansprüche 1 bis 8, wobei das monoklonale Antikörper Panel in Multiplex-Antikörper-Arrays umfassend fluoreszierende Antikörperkonjugierte Kügelchen oder über Chemilumineszenz Nachweis oder über Nachweise, die keine Markierung verwenden, bevorzugt Massenspektrometrie durchmustert wird.

10. Ein Verfahren nach einem der Ansprüche 8 bis 9, wobei die Dichte des Arrays zwischen 10-1.000/cm² oder zwischen 1.000-1.000.000/cm² ist.

11. Das Verfahren nach einem der vorhergehenden Ansprüche, wobei die Identität des verwandten Antigens, das spezifisch an den Antikörper oder Derivat davon gebunden ist, über gekoppelte Chromatographie und/oder durch elektrisches Feld vermittelte Trennungsverfahren- Massenspektrometrie oder direkte Peptidsequenzierungsverfahren bestimmt wird.

12. Das Verfahren nach einem der vorhergehenden Ansprüche, wobei die biologische Analyt-Probe aus der Gruppe bestehend aus Plasma, Serum, Urin, Körperflüssigkeiten, Zelllysaten, Gewebeextrakten humanen Ursprungs ausgewählt ist, irgendeinem Gemisch, das immunogene Metabolite und/oder immunogene Proteine oder Peptide enthält oder eine Kombination davon.

13. Ein Verfahren nach einem der vorhergehenden Ansprüche, wobei der Prozess einen oder mehrere Mikrofluidikchips oder miniaturisierte Gesamtanalysesysteme (µTAS) involviert.

14. Ein Verfahren nach einem der vorhergehenden Ansprüche, wobei vor der Immunisierung der Analyt an einen Träger, bevorzugt Albumin, Polylysin oder Keyhole-Limpet-Hämocyanin konjugiert wird.

15. Das Verfahren nach einem der vorhergehenden Ansprüche, wobei das Antikörper-Derivat ein Antikörper-Fragment, bevorzugt ausgewählt aus Fab, Fab', CDR und einkettige Antikörper (ScFv) ist, ferner bevorzugt ist ein humaner oder humanisierter Antikörper oder Fragment davon.

16. Das Verfahren nach einem der vorhergehenden Ansprüche, wobei die biologische Analyt-Probe von einem gesunden humanen Subjekt stammt.

17. Das Verfahren nach einem der vorhergehenden Ansprüche, wobei das Panel monoklonale Antikörper oder Derivate davon umfasst, die aus biologischen Analyt-Proben von verschiedenen humanen Subjekten, bevorzugt von mehreren gesunden Subjekten oder von mehreren gesunden und erkrankten Subjekten hergestellt werden.

18. Das Verfahren nach einem der vorhergehenden Ansprüche, wobei unterschiedliche Klassen von Antigenen, die in der Probe vorhanden sind, getrennt werden, und getrennte Immunisierungen mit jedem der Klassen durchgeführt werden.

19. Das Verfahren nach einem der vorhergehenden Ansprüche, wobei das Panel eine Vielzahl monoklonaler Antikörper, die Hybridomen und/oder Derivate davon herstellen, umfasst, die in getrennten Behältern angeordnet sind.

## Revendications

1. Méthode pour identifier des antigènes de sang humain dans un échantillon biologique d'analytes, comprenant les étapes suivantes :
a) enrichissement de l'échantillon d'analytes, selon lequel ledit échantillon est traité par partition en utilisant une technologie de séparation dans des conditions permettant la déplétion d'au moins 2 à 22 des protéines humaines les plus abondantes représentées dans le plasma à un niveau de concentration supérieur à 1mg/ml, tout en conservant les protéines faiblement abondantes présentes dans le sang humain normal, et selon lequel l'échantillon déplété comprend moins de 5% des protéines de sérum humain total ;
b) de manière facultative, séparation de l'échantillon d'analytes enrichi en des classes (pools) spécifiques d'analytes, chaque classe (pool) d'analytes contenant des éléments ayant au moins une caractéristique physique, chimique, immunochimique ou biochimique commune ;
c) immunisation d'un sujet mammifère non-humain avec soit (i) l'échantillon d'analytes enrichi de l'étape a) ou un aliquot ou une dilution de celui-ci, soit avec (ii) un pool d'analytes selon l'étape b) ;
d) génération d'un panel d'anticorps monoclonaux, de dérivés de ceux-ci, ou de cellules d'hybridomes correspondantes à partir des sujets mammifères non-humains immunisés ;
e) sélection d'anticorps de d) qui lient des antigènes dudit échantillon d'analytes ;
f) purification d'antigènes à partir de l'échantillon d'analytes par affinité d'enrichissement en utilisant des anticorps monoclonaux ou dérivés de ceux-ci, sélectionnés en e) ; et
g) détermination de l'identité desdites antigènes humains.

2. Méthode selon la revendication 1, dans laquelle ladite étape de traitement a) comprend une chromatographie d'affinité de ligand organique, une chromatographie d'échange d'ions, une chromatographie d'interaction hydrophobe, une chromatographie d'interaction hydrophile, une chromatographie d'exclusion de taille, un Chromatofocusing, ou un isoelectrofocusing, ou une combinaison de ceux-ci.

3. Méthode selon l'une quelconque des revendications précédentes, dans laquelle ledit mammifère non-humain est un rongeur ou un lapin.

4. Méthode selon l'une quelconque des revendications précédentes, dans laquelle les éléments du pool d'analytes de l'étape b) partagent des capacités d'affinité de liaison et/ou sont homologues dans leur séquence peptidique.

5. Méthode selon l'une quelconque des revendications précédentes, dans laquelle l'étape e) comprend la mise en contact dudit panel d'anticorps monoclonaux ou de dérivés de ceux-ci, tels que définis ou susceptibles d'être obtenus par une méthode selon l'une des revendications 1 à 4, avec ledit échantillon d'analytes, dans des conditions permettant la réalisation d'une réaction antigène-anticorps, et l'identification d'un ou de plusieurs d'anticorps monoclonaux ou dérivés de ceux-ci, dudit panel, qui forment des complexes anticorps-antigène avec ledit échantillon.

6. Méthode selon l'une quelconque des revendications précédentes, dans laquelle l'étape e) comprend l'identification d'anticorps qui sont spécifiques d'une condition ou maladie particulière, la méthode comprenant la mise en contact d'un échantillon biologique d'analytes provenant d'un mammifère présentant ladite condition ou maladie avec tout ou une portion d'un panel d'anticorps monoclonaux ou de dérivés de ceux-ci, tels que définis ou susceptibles d'être obtenus par une méthode selon l'une des revendications 1 à 4, dans des conditions permettant la réalisation d'une réaction antigène-anticorps, et l'identification d'un ou de plusieurs d'anticorps monoclonaux ou dérivés de ceux-ci, dudit panel, qui forment des complexes anticorps-antigène avec ledit échantillon.

7. Méthode selon l'une des revendications 1 à 6, dans laquelle ledit panel d'anticorps monoclonaux est testé par test ELISA.

8. Méthode selon l'une des revendications 1 à 7, dans laquelle ledit panel d'anticorps monoclonaux est immobilisé sur une surface solide, de préférence une surface en verre, une surface en silice, une surface plastique ou une surface en métal, et testé comme un microarray, ou dans laquelle ledit panel d'anticorps monoclonaux est immobilisé sur tout gel, ou membrane, de préférence des membranes lipidiques, pour le test.

9. Méthode selon l'une des revendications 1 à 8, dans laquelle ledit panel d'anticorps monoclonaux est criblé dans des arrays d'anticorps multiplexes comprenant des billes conjuguées à des anticorps fluorescents, ou par test chimioluminescent, ou par des tests n'utilisant pas de marqueur, de préférence la spectrométrie de masse.

10. Méthode selon l'une des revendications 8 à 9, dans laquelle la densité de l'array est entre 10-1,000/cm², ou entre 1,000-1,000,000/cm².

11. Méthode selon l'une quelconque des revendications précédentes, dans laquelle l'identité de l'antigène spécifiquement lié au dit anticorps, ou dérivé de celui-ci, est déterminée par chromatographie de liaison et/ou par des méthodes de séparation médiée par champ électrique - spectrométrie de masse ou des méthodes de séquençage direct de peptide.

12. Méthode selon l'une quelconque des revendications précédentes, dans laquelle l'échantillon biologique d'analytes est choisi dans le groupe constitué de plasma, sérum, urine, fluides corporels, lysats cellulaires, extraits tissulaires d'origine humaine ; tout mélange contenant des métabolites immunogènes et/ou des peptides ou protéines immunogènes ; ou une combinaison de ceux-ci.

13. Méthode selon l'une quelconque des revendications précédentes, dans laquelle la méthode implique une ou plusieurs puces microfluidiques ou micro système d'analyse totale (µTAS).

14. Méthode selon l'une quelconque des revendications précédentes, dans laquelle, avant l'immunisation, l'analyte est conjugué à un support, de préférence l'albumine, la polylysine, ou l'hémocyanine de keyhole limpet (KLH).

15. Méthode selon l'une quelconque des revendications précédentes, dans laquelle le dérivé d'anticorps est un fragment d'anticorps, de préférence choisi parmi Fab, Fab', CDR et les anticorps simple chaine (ScFv), plus préférentiellement un anticorps humain ou humanisé ou un fragment de celui-ci.

16. Méthode selon l'une quelconque des revendications précédentes, dans laquelle l'échantillon biologique d'analytes est dérivé d'un sujet humain sain.

17. Méthode selon l'une quelconque des revendications précédentes, dans laquelle le panel comprend des anticorps monoclonaux ou des dérivés de ceux-ci qui sont produits à partir d'échantillons biologiques d'analytes provenant de sujets mammifères différents, de préférence de plusieurs sujets sains ou de plusieurs sujets sains et pathologiques.

18. Méthode selon l'une quelconque des revendications précédentes, dans laquelle différentes classes d'antigènes présents dans l'échantillon sont séparées, et des immunisations séparées sont réalisées avec chacune de ces classes.

19. Méthode selon l'une quelconque des revendications précédentes, dans laquelle le panel comprend une pluralité d'anticorps monoclonaux, d'hybridomes producteurs, ou de dérivés de ceux-ci, qui sont disposés dans des containeurs séparés.
